# EUROPEAN PATENT SPECIFICATION

(11) **EP 1 430 918 B1**
(45) Date of publication and mention of the grant of the patent: **14.05.2008**
(21) Application number: 03405885.9
(22) Date of filing: 11.12.2003
(51) Int. Cl.: A61M 1/06, A61B 5/00

(54) **Use of a breast pump**
Verwendung einer Brustpumpe
Utilisation d'une pompe à lait

(30) Priority: 20.12.2002 CH 21882002; 27.03.2003 US 401138; 17.07.2003 US 621490
(43) Date of publication of application: 23.06.2004
(73) Proprietor: Medela Holding AG, 6340 Baar (CH)
(72) Inventor: Kent, Jacqueline Coral, Nedlands, WA 6009 (AU); Mitoulas, Leon Robert, Osborne Park, WA 6017 (AU); Ramsay, Donna Tracy, Forrestfield, WA 6058 (AU); Hartmann, Peter Edwin, Gooseberry Hill, WA 6076 (AU); Cregan, Mark Derek, Doubleview, WA 6018 (AU); Sadleir, Rosalind Jane, Gainsville, FL 32601-4352 (US)
(74) Representative: Clerc, Natalia

(56) References cited:
- EP-A- 0 116 186
- WO-A-01/47577
- WO-A-01/74149
- WO-A-03/028616
- CA-A- 2 340 851
- US-A- 5 827 191
- HARTMANN PETER AND CREGAN MARK: "Lactogenesis and the effects of insulin-dependent diabetes mellitus and prematurity" 2 April 2001 (2001-04-02) , AMERICAN SOCIETY FOR NUTRITIONAL SCIENCES XP002270836 * page 3017S, right-hand column, line 21 - line 36 *
- DALY S E J ET AL: "Degree of breast emptying explains change in the fat content, but not fatty acid composition, of human milk" EXPERIMENTAL PHYSIOLOGY, XP008030117

## Description

### Technical field

The invention relates to a system and to a process for detecting a milk surge in a mother's breast and to the use of a breast pump for detecting a milk surge.

### Prior Art

The human breast has mammary glands which form balloon-like structures, so-called alveoli. The alveoli are connected to one another via milk ducts which lead to the nipple. The alveoli are enclosed by myoepithelial cells which contract under the influence of oxytocin. A sphincter at the end of the nipple, however, prevents the breast milk from flowing out.

If a baby then begins to suck at the breast, this is detected by mechanicoreceptors in the breast and a corresponding signal is transmitted to the mother's brain. Influenced by emotions, experiences and other external influences, the signal passes to the hypophysis which thus releases oxytocin.

The contraction of the myoepithelial cells triggered as a result leads to the alveoli deflating, as a result of which the milk ducts are widened. If the nipple sphincter is then opened by pressure, heat or other external factors, the milk can flow out. This contraction is referred to as the milk surge.

The prior art discloses breast pumps which are likewise able to express milk from the mother's breast. In particular WO 01/47577 discloses a milk pump which imitates the sucking rhythm of a baby and thus allows milk to be expressed from the breast in as natural a manner as possible.

EP 0'116'186 discloses a manually operated breast pump with a collecting cylinder. The amount of milk in the cylinder can be visually determined by means of a scale.

Breast-feeding a baby, however, is not always straightforward. The causes of problems may stem from the baby or the mother or from both. It is often difficult, however, to determine the causes precisely. Expressing milk by means of a breast pump can also be problematic for some mothers. For the manufacturers of breast pumps, it is thus important to understand breast-feeding as precisely as possible and to utilize this knowledge in the design of breast pumps. It has been found that essential information for analyzing breast-feeding problems and for optimizing breast pumps may be derived from the mother's milk surge.

Hartmann Peter and Cregan Mark: "Lactogenesis and the effects of insulin-dependent diabetes mellitus and prematurity", 2 April 2001 (2001-04-02), American Society for nutritional sciences, discusses the initiation of lactation by the mother. This initiation has to be synchronized to the delivery of the infant, permitting the newborn to change from continuous nourishment from the umbilical cord to comparable life support from its mother's breast. They therefore watch the change in concentration of lactose from birth to 7 days postpartum .

It is another aspect of breast-feeding, that the volume of milk consumed by a baby is determined in order to make sure, that the baby is well fed. The simplest, however also a not very efficient way to do so is to weigh the baby before and after the feeding session.

US 5'827'191 therefore discloses a method for monitoring a volume of milk during breast feeding, the method utilizing an elastic nipple shaped cover applied over a nipple area of a woman's breast. The cover has holes positioned above the nipple area for passage of milk to the baby's mouth. A micro measurement volume sensor is located in a space between the nipple and the elastic cover holes to measure the volume of milk flowing therethrough.

WO 01/54488 also discloses an apparatus for determining the amount of human milk supplied to a feeding baby during a breast-feeding session. A flowmeter is used to measure the milk supply.

WO 01/47577 discloses a programmable pump that can record data relating to its use and operation.

### Description of the invention

It is an object of the invention to provide a system and a process which allows a milk surge to be detected in a straightforward manner.

This object is achieved by a system and a process having the features of patent claims 1 and 9, respectively.

It is another object of the invention to provide a system and a process which allows taking samples for milk analysis and/or individual use without interfering with a determination of the milk quantity.

This object is achieved by a system and a process having the features of patent claims 7 and 13, respectively.

In the process according to the invention, the milk is expressed into at least one collecting container and the quantity of milk expressed is determined as a function of time. If a milk surge takes place, then the quantity of milk detected increases abruptly. It is thus easy to detect the milk surge.

The quantity of milk is preferably weighed. However, other determining methods, for example volume measurements, are also possible.

The change in the quantity of milk over time is preferably directly determined or calculated. This allows the point in time and also the intensity of the milk surge to better be detected.

The measurement results and measurement curves obtained in this way can be evaluated, and it is possible to draw conclusions about the behavior of the corresponding test individual in response to various external and internal influences. Application areas for the system and process according to the invention are, for example, research, in order to obtain knowledge about the breast-feeding behavior of babies and mothers. They may also be used, however, in hospitals or for advising mothers, in order to resolve breast-feeding or expressing problems. The results may also be used in product development, for the purpose of optimizing breast pumps.

In a variant of the process, the milk is collected in several containers, wherein the quantity of milk expressed is still determined as a function of time. If the quantity of milk is determined by weighing, the containers are preferably placed on the same balance. This enables splitting of milk collection whilst not interfering with the continuous collection of weight data. Since the milk collection is split, the milk samples can be individually analyzed and/or used.

Further advantageous variants and embodiments can be gathered from the dependent patent claims.

### Brief description of the drawing

The subject matter of the invention is explained hereinbelow with reference to preferred exemplary embodiments illustrated in the attached drawing, in which:
- Figure 1: shows a schematic illustration of the system according to the invention;
- Figure 2: shows a measurement curve obtained by means of the process according to the invention and a calculated first derivative of the measurement curve;
- Figure 3: shows a schematic illustration of the system according to a second embodiment of the invention and
- Figure 4: shows a schematic illustration of the system according to a third embodiment of the invention.

### Methods of implementing the invention

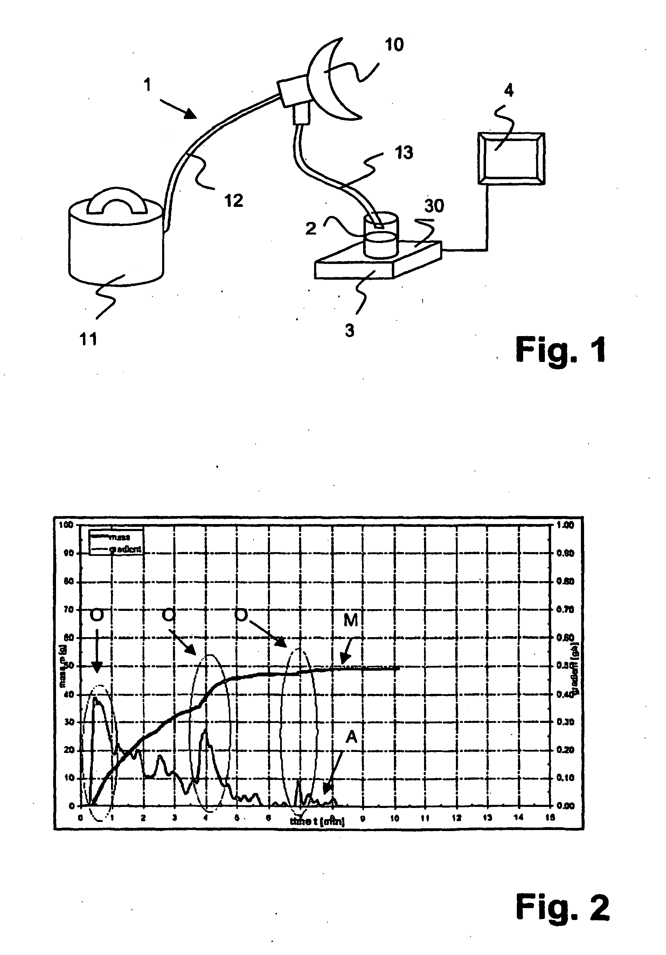

The system of the invention according to Figure 1 has a breast pump 1, with at least one breast shield 10 for expressing the milk from a human breast, at least one collecting container 2, for receiving the milk expressed, and a unit with a measuring means 3 and an evaluating means 4, by means of which a quantity of milk received in the collecting container 2 is determined as a function of time.

The measuring means 3 serves for determining the weight or the change in weight of the breast milk located in the collecting container 2. In this preferred exemplary embodiment, this measuring means 3 is a balance, preferably an electromechanical balance with a bearing surface 30, on which the collecting container 2 is arranged.

The balance 3 is electronically connected to the evaluating means 4, which is preferably a computing system, in particular a computer, in order to transmit the measured values from the balance 3 to the computer 4. It is also possible, however, for the computer 4 and balance 3 to be integrated in a single machine. The measured values may be transmitted at defined time intervals or in a continuous manner.

In the evaluating means 4, the measured values and/or the change therein are illustrated as a function of time. Figure 2 illustrates a corresponding measurement curve M and the derivative A thereof as a function of time. The y-axis shows the time in minutes, and the x-axis shows the mass in grams. The ovals O in figure 2 indicate the point in time of a milk surge. As can be seen, the flow of milk increases during a milk surge; the measurement curve M rises more rapidly. In the derivative A, the milk surges appear in the form of peaks and are thus even easier to make out. It can also be seen from the two curves M, A that the milk surge need not always have the same intensity. These curves can be evaluated and the results used for a variety of different purposes mentioned above.

Figure 1 illustrates a table-top model of a breast pump. This means that the pumping unit is arranged in a housing 11 and is connected, by means of a negative-pressure tube 12, to the breast shield 10, in order for the negative pressure which is necessary for the flow of milk to be achieved therein. The breast shield 10 is connected to the collecting container 2 via a connecting tube 13, with the result that the breast milk can pass through this tube 13 into the container 2, for example a glass or a bottle. This apparatus has the advantage that the mother can move about during the test without falsifying the measurement result.

It is also possible, however, to use a breast pump in which the collecting container 2 is arranged on the breast shield 10. Here the important factor is for it to be possible to detect the behavior of the flow of milk over time.

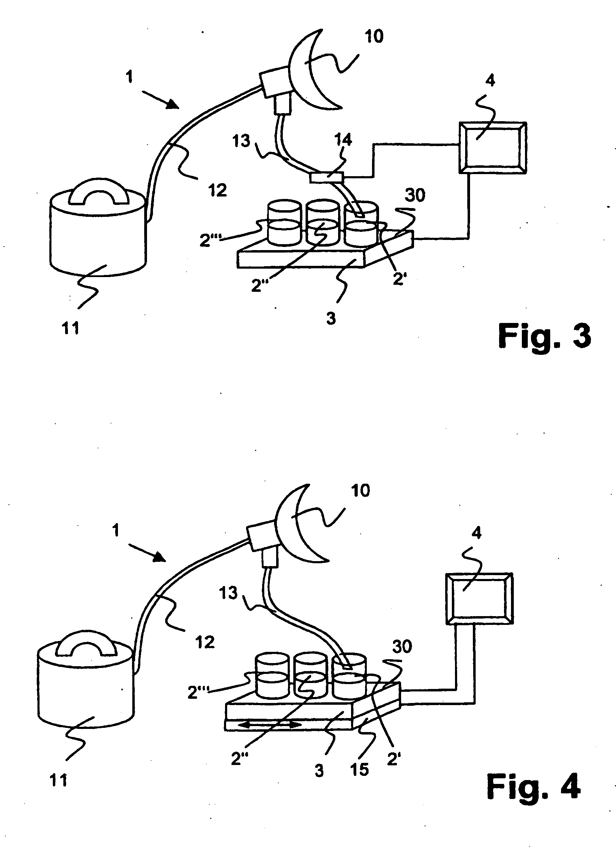

Figure 3 shows a second embodiment of the invention. Instead of one single collecting container 2, several containers 2', 2'', 2''' are used. The containers 2', 2'', 2''' can have the same or different volumes. The number of containers 2', 2'', 2''' depends on the kind of analysis to be made. The three containers shown in figure 3 are therefore only an example. Preferably the containers 2', 2'', 2''' are all connected to the same measuring means 3, which can be any of the measuring means mentioned above. In the example shown in figure 3, the measuring means 3 is again a balance, so that the containers 2', 2'', 2''' are placed on this balance 3.

The connecting tube 13 is preferably coupled with first moving means 14 for moving the tube 13 from a first to a second of said containers 2', 2'', 2'''. The tube 13 is moved to the next container 2', 2'', 2'" after a predetermined event. It is preferably moved automatically, the means being preferably controlled by the evaluating means 4. It is also possible to connect the evaluation means and the moving means to a separate, but common control means. This event is preferably a time period passed, so that the connecting tube 13 is moved after a set time point. The time point can always be the same or it can change depending on the container to be filled. The event can also be a predetermined quantity of milk collected in one of the containers 2', 2'', 2''' or it can be something else.

The milk collected in the several containers 2', 2'', 2''' can be analyzed and also used individually. For example, as milk is removed from the breast the fat content of the milk increases and this system allows to track that increase.

This technique is extremely beneficial for mothers of premature infants for whom the energy density of milk is very important. The fat is responsible for approximately 50% of the energy in milk therefore, collecting the milk in fractions will provide volumes of milk with different energy densities. These can then be used individually or certain fractions can even be mixed to provide milk of specific energy density - an energy density best suited to the infant's needs.

Figure 4 shows third embodiment of the inventive system. Here, the measuring means 3, i.e. in this case the balance, is moved by second moving means 15 in order to fill the different containers 2', 2'', 2'''. This moving means 15, which can for example be a motor-driven moving table, where the balance is being placed on, is preferably connected to the evaluation and control means 4.

### List of designations

- 1: Breast pump
- 10: Breast shield
- 11: Housing
- 12: Negative-pressure tube
- 13: Connecting tube
- 14: moving means
- 15: second means

- 2: Collecting container
- 2': Collecting container
- 2'': Collecting container
- 2''': Collecting container

- 3: Measuring means
- 30: Bearing surface

- 4: Evaluating means

- M: Measurement curve
- A: Derivative
- O: Oval

## Claims

1. A system for detecting a milk surge in a mother's breast, the system having a breast pump (1) with a breast shield (10) for expressing milk from the breast, at least one collecting container (2), for receiving the milk expressed, and a unit (3,4) by means of which a quantity of milk received in the at least one collecting container (2) is determined as a function of time while the milk is expressed, wherein the unit (3,4) has a measuring means (3), for measuring the quantity of milk located in the at least one collecting container (2), and an evaluating means (4) by means of which the quantity of milk measured is evaluated as a function of time.

2. The system as claimed in claim 1, wherein the measuring means (3) is a balance.

3. The system as claimed in claim 2, wherein the balance (30) is an electromechanical balance with a bearing surface for the at least one collecting container (2).

4. The system as claimed in claim 1, wherein the evaluating means (4) is a computing system, in particular a computer.

5. The system as claimed in claim 1, wherein the at least one collecting container (2) is connected to the breast shield(10) via a connecting tube (13).

6. The system as claimed in claim 1, wherein the system comprises several collecting containers (2,2', 2" , 2"') being connected with said unit (3,4) for determining the quantity of milk

7. The system as claimed in claim 6 and claim 2, wherein said collecting containers (2, 2', 2 " , 2"') are arranged on said balance.

8. The system as claimed in claim 6 and claim 5, wherein the system comprises moving means (14) for moving said connecting tube (13) from one of said collecting containers to another of said collecting containers (2, 2', 2'', 2'").

9. A process for detecting a milk surge in a mother's breast, milk being expressed from the breast into at least one collecting container (2), and the quantity of milk expressed being determined as a function of time while the milk is expressed, wherein a unit (3,4) determines a quantity of milk received in at least one collecting container (2) as a function of time while the milk is expressed, and wherein the unit (3,4) has a measuring means (3), for measuring the quantity of milk located in the at least one collecting container (2), and an evaluating means (4) by means of which the quantity of milk measured is evaluated as a function of time.

10. The process as claimed in claim 9, wherein the weight of the quantity of milk expressed is determined as a function of time.

11. The process as claimed in claim 9, wherein the change in weight of the quantity of milk expressed is determined as a function of time.

12. The process as claimed in claim 9, wherein, in order to express the milk, use is made of a breast pump (1) with a breast shield (10), and wherein the milk expressed is directed from the breast shield (10) into the at least one collecting container (2) via a connecting tube (13).

13. The process as claimed in claim 9, wherein the milk is collected in several collecting containers (2, 2', 2" , 2' ' '), wherein the collecting containers (2, 2' , 2' ' , 2"'), are filled one after the other dependent on a predetermined event.

14. The process as claimed in claim 13, wherein the predetermined event is the arrival of a set time point.

15. Use of a breast pump(1) according to any of claims 1 - 8 for detecting a milk surge in a mother's breast, milk being expressed from the breast into at least one collecting container (2) by means of the breast pump (1), and the quantity of milk expressed being determined as a function of time while the milk is expressed.

## Patentansprüche

1. System zur Detektion eines Milcheinschusses in einer Mutterbrust, wobei das System eine Brustpumpe (1) mit einer Absaughaube (10) zur Absaugung von Milch aus der Brust, mindestens einen Auffangbehälter (2) zur Aufnahme der abgesaugten Milch sowie eine Einheit (3, 4) zur Bestimmung einer in dem mindestens einen Auffangbehälter (2) aufgenommenen Milchmenge als eine Funktion der Zeit während die Milch abgesaugt wird, wobei die Einheit (3, 4) ein Messmittel (3) zur Messung der Milchmenge, die sich in dem mindestens einen Auffangbehälter (2) befindet, aufweist, und ein Auswertemittel (4) zur Auswertung der gemessenen Milchmenge als eine Funktion der Zeit.

2. System nach Anspruch 1, wobei das Messmittel (3) eine Waage ist.

3. System nach Anspruch 2, wobei die Waage (3) eine elektromechanische Waage mit einer Auflagefläche (30) für den mindestens einen Auffangbehälter (2) ist.

4. System nach Anspruch 1, wobei das Auswertemittel (4) ein Rechnersystem, insbesondere ein Computer, ist.

5. System nach Anspruch 1, wobei der mindestens eine Auffangbehälter (2) über einen Verbindungsschlauch (13) mit der Absaughaube (10) verbunden ist.

6. System nach Anspruch 1, wobei das System mehrere Auffangbehälter (2, 2', 2" , 2"') aufweist, welche mit der besagten Einheit (3, 4) zur Bestimmung der Milchmenge verbunden sind.

7. System nach Anspruch 6 und Anspruch 2, wobei die besagten Auffangbehälter (2, 2', 2", 2"') auf der besagten Waage angeordnet sind.

8. System nach Anspruch 6 und Anspruch 5, wobei das System ein Bewegungsmittel (14) aufweist, um den besagten Verbindungsschlauch (13) von einem der besagten Auffangbehälter zu einem anderen der besagten Auffangbehälter (2, 2' , 2" , 2"') zu bewegen.

9. Verfahren zur Detektion eines Milcheinschusses in einer Mutterbrust, wobei Milch aus der Brust in mindestens einen Auffangbehälter (2) abgesaugt wird und wobei die abgesaugte Milchmenge als eine Funktion der Zeit bestimmt wird, wobei eine Einheit (3, 4) eine Milchmenge, die in mindestens einem Auffangbehälter (2) aufgenommen wird, als eine Funktion der Zeit bestimmt wird, und wobei die Einheit (3, 4) ein Messmittel (3) zur Messung der Milchmenge, die sich in dem mindestens einen Auffangbehälter (2) befindet, und ein Auswertemittel (4), mittels dessen die gemessene Milchmenge als eine Funktion der Zeit ausgewertet wird, aufweist.

10. Verfahren nach Anspruch 9, wobei das Gewicht der abgesaugten Milchmenge als eine Funktion der Zeit bestimmt wird.

11. Verfahren nach Anspruch 9, wobei die Gewichtsveränderung der abgesaugten Milchmenge als eine Funktion der Zeit bestimmt wird.

12. Verfahren nach Anspruch 9, wobei zur Absaugung der Milch eine Brustpumpe (1) mit einer Absaughaube (10) verwendet wird und dass die abgesaugte Milch über einen Verbindungsschlauch (13) von der Absaughaube (10) in den mindestens einen Auffangbehälter (2) geleitet wird.

13. Verfahren nach Anspruch 9, wobei die Milch in mehreren Auffangbehältern (2, 2', 2" , 2" ') gesammelt wird, wobei die Auffangbehälter (2, 2', 2" , 2" ') abhängig von einem vorbestimmten Ereignis einer nach dem anderen aufgefüllt werden.

14. Verfahren nach Anspruch 13, wobei das vorbestimmte Ereignis das Erreichen eines vorprogrammierten Zeitpunktes ist.

15. Verwendung einer Brustpumpe (1) nach einem der Ansprüche 1-8 zur Detektion eines Milcheinschusses in einer Mutterbrust, wobei mittels der Brustpumpe (1) Milch aus der Brust in mindestens einen Auffangbehälter (2) hinein abgesaugt wird und wobei die abgesaugte Milchmenge als eine Funktion der Zeit bestimmt wird, während die Milch abgesaugt wird.

## Revendications

1. Système pour détecter un afflux de lait dans le sein d'une mère, le système ayant un tire-lait (1) avec un coussin protecteur (10) pour tirer du lait d'un sein, au moins un récipient collecteur (2) pour recevoir le lait tiré, et une unité (3, 4) au moyen de laquelle une certaine quantité de lait reçue dans l'au moins un récipient collecteur (2) est déterminée en fonction du temps pris pour tirer le lait, l'unité (3, 4) ayant un moyen de mesure (3) pour mesurer la quantité de lait se trouvant dans l'au moins un récipient collecteur (2), et un moyen d'évaluation (4) au moyen duquel la quantité de lait mesurée est évaluée en fonction du temps.

2. Système selon la revendication 1, dans lequel le moyen de mesure (3) est une balance.

3. Système selon la revendication 2, dans lequel la balance (30) est une balance électromécanique, avec une surface de support pour l'au moins un récipient collecteur (2).

4. Système selon la revendication 1, dans lequel le moyen d'évaluation (4) est un système informatique, en particulier un ordinateur.

5. Système selon la revendication 1, dans lequel l'au moins un récipient collecteur (2) est connecté au coussin protecteur (10) par le biais d'un tube de connexion (13).

6. Système selon la revendication 1, dans lequel le système comprend plusieurs récipients collecteurs (2, 2', 2", 2"') connectés à ladite unité (3, 4) pour déterminer la quantité de lait.

7. Système selon la revendication 6 et la revendication 2, dans lequel lesdits récipients collecteurs (2, 2', 2", 2"') sont arrangés sur ladite balance.

8. Système selon la revendication 6 et la revendication 5, dans lequel le système comprend des moyens de déplacement (14) pour déplacer ledit tube de connexion (13) d'un desdits récipients collecteurs à un autre desdits récipients collecteurs (2, 2', 2", 2"').

9. Procédé pour détecter un afflux de lait dans le sein d'une mère, du lait étant tiré du sein dans au moins un récipient collecteur (2), et la quantité de lait tirée étant déterminée en fonction du temps pendant lequel le lait est tiré, dans lequel une unité (3, 4) détermine une certaine quantité de lait reçue dans au moins un récipient collecteur (2) en fonction du temps pris pour tirer le lait, et dans lequel l'unité (3, 4) a un moyen de mesure (3) pour mesurer la quantité de lait se trouvant dans l'au moins un récipient collecteur (2), et un moyen d'évaluation (4) au moyen duquel la quantité de lait mesurée est évaluée en fonction du temps.

10. Procédé selon la revendication 9, dans lequel le poids de la quantité de lait tirée est déterminé en fonction du temps.

11. Procédé selon la revendication 9, dans lequel le changement de poids de la quantité de lait tirée est déterminé en fonction du temps.

12. Procédé selon la revendication 9, dans lequel, afin de tirer du lait, on utilise un tire-lait (1) avec un coussin protecteur (10), et dans lequel le lait tiré est dirigé depuis le coussin protecteur (10) dans l'au moins un récipient collecteur (2) par le biais d'un tube de connexion (13).

13. Procédé selon la revendication 9, dans lequel le lait est recueilli dans différents récipients collecteurs (2, 2', 2", 2"'), les récipients collecteurs (2, 2', 2", 2"') étant remplis les uns après les autres en fonction d'un événement prédéterminé.

14. Procédé selon la revendication 13, dans lequel l'événement prédéterminé est l'arrivée d'un instant donné.

15. Utilisation d'un tire-lait (1) selon l'une quelconque des revendications 1 à 8, pour détecter un afflux de lait dans le sein d'une mère, du lait étant tiré du sein dans au moins un récipient collecteur (2) au moyen du tire-lait (1), et la quantité de lait tirée étant déterminée en fonction du temps pris pour tirer le lait.
